# EUROPEAN PATENT APPLICATION

(11) **EP 1 457 166 A1**
(43) Date of publication of application: **15.09.2004**
(21) Application number: 04425084.3
(22) Date of filing: 10.02.2004
(51) Int. Cl.: A61C 8/00

(54) **Micronail and method for dental use**

(30) Priority: 13.03.2003 IT BS20030028
(71) Applicant: Physioplant S.r.l., 25030 Roncedelle (Brescia) (IT)
(72) Inventor: Rizzo, Rosario, 25030 Roncadelle (Brescia) (IT)
(74) Representative: Sangiacomo, Fulvia

(57) **Abstract**

The invention refers to a method for fastening a protective membrane for dental use to a jaw bone and consists, first of all, in fitting into the bone a micronail having a head with at least one means of retention and, then, the fastening of the membrane to said means of retention on the head of the micronail. The head (12) of the micronail features means of retention for fastening a membrane, directly or with the aid of a retention element, after the micronail has been fitted into the jaw bone involved.

## Description

### Field of the Invention

The present invention refers to a micronail and a method for its use especially in the field of dentistry.

### State of the Art

Some dental operations or treatments can cause or leave a cavity in the treated jaw bone or within tooth alveoli. To subsequently favour bone growth in that area, the cavity is normally covered with a membrane intended to protect the tissue forming underneath and prevent the colonisation of the site by other tissues. To ensure its stability and immobility, the membrane is normally fastened to the jaw bone using microscrews, micronails or similar.

The use of microscrews requires making, at the same time, a number of holes through the protective membrane and in the jaw bone in which to tighten the screws so their heads fasten and retain the membrane. When micronails are used, after crossing the membrane, these are pushed into the bone and again fasten the membrane with their heads. Sometimes, when the bone is very hard, the use of micronails also requires holes to be made beforehand.

In any case, microscrews or micronails are fitted after the membrane has been distended on the cavity to be closed. Nevertheless, because the membrane is a non-transparent material, it is obvious that the holes for the microscrews or fitting the micronails made by passing through the membrane are substantially made blind, meaning without clearly seeing where the operation will finish and with the risk of placing the means of retention in incorrect positions and/or positions that interfere with tooth roots, already implanted dental implants or other parts.

### Objects and Summary of the invention

The object of this invention is to effectively overcome the drawbacks and risks produced by adopting a new and original means of retention to be first of all implanted in the bone involved in order to subsequently fasten the protective membrane to be secured.

With the means of retention of the invention, the sequence is substantially reversed of the fitting phases of a membrane straddling and protecting a cavity in a bone, such as the jaw bone. In fact, whereas with the procedure followed so far, the membrane is first distended on the cavity and then secured to the bone, now the means of retention are first of all implanted in the bone, with total visibility of the operating area, and then the membrane is fastened to the means of retention.

The object purpose and evident advantages that ensue are achieved, according to the invention, with a method of retention to a jaw bone of a protective membrane for dental use comprising, first, the fastening into a jaw bone of a micronail having a head with at least one means of retention and, then, the fastening of the membrane to said means of retention on the micronail head.

Always according to the invention, a micronail for the implementation of such fastening method includes a shank and a head, where the head features means of retention for fastening a membrane, directly or with the aid of a retention element, after the micronail has been fitted into the bone involved.

According to one example of embodiment, the means of retention can consist of a part of the head of the micronail on which to engage the membrane, and a retention element for circling and covering the head and superimposing the membrane.

As means of retention, the head of the micronail can be provided with a ring-shaped groove at the base and a portion of the top on which to engage the membrane, and also have a retention element circling or enclosing the head and the membrane resting on it.

And again, the means of retention on the head of the micronail can consist of a tang that can be folded, after perforating and crossing the membrane, and if necessary fastened by means of a retention element fitted on the membrane and head.

In any case, the retention element can take the form of a ring or cap with dimensions compatible with the head and the membrane resting on this.

According to another example of embodiment, the head of the micronail can be provided, as a means of retention for the membrane, with a fastening system consisting of a press stud, always and in any case in compliance with the procedure which envisages first of all the fitting of the micronail into the bone and, afterwards the fastening of the membrane to the micronail.

According to another example of embodiment, the head of the micronail can present a perforating portion both in association with and not in association with a groove at the base of the head. In this case, in the assembly, the perforating portion, after the micronail has been fitted in the bone, acts as a means for perforating the membrane and guiding this towards the ring-shaped groove at the base of the head. This groove is intended to receive and withhold, with the aid of a retention ring fitted on the head of the micronail, part of the membrane around the hole produced by such perforation. The perforating portion can, if necessary, be removed by means of a suitable tool, leaving the membrane fastened to the head of the micronails previously fitted into the bone.

### Brief description of drawings

The invention will be illustrated in more detail in the following description with reference made to the attached approximate, but not limitative, drawings, in which:
Fig. 1 shows an enlarged view of a micronail;
Fig. 1a shows an enlarged view of a micronail with perforating portion;
Fig. 2 schematically shows micronails according to Fig. 2 fitted into a bone and with the membrane being fitted, and
Fig. 3 shows the diagram of Fig. 2, but after the fitting of the membrane to the micronails.

### Detailed description of the invention

The micronail shown is made from a biocompatible material and includes a pointed shank 11 and a head 12, which can be cylindrical or of other shape. Along the shank 11, a tapered intermediate area 13 can be obtained and, next to head 12, a supporting flange 14.

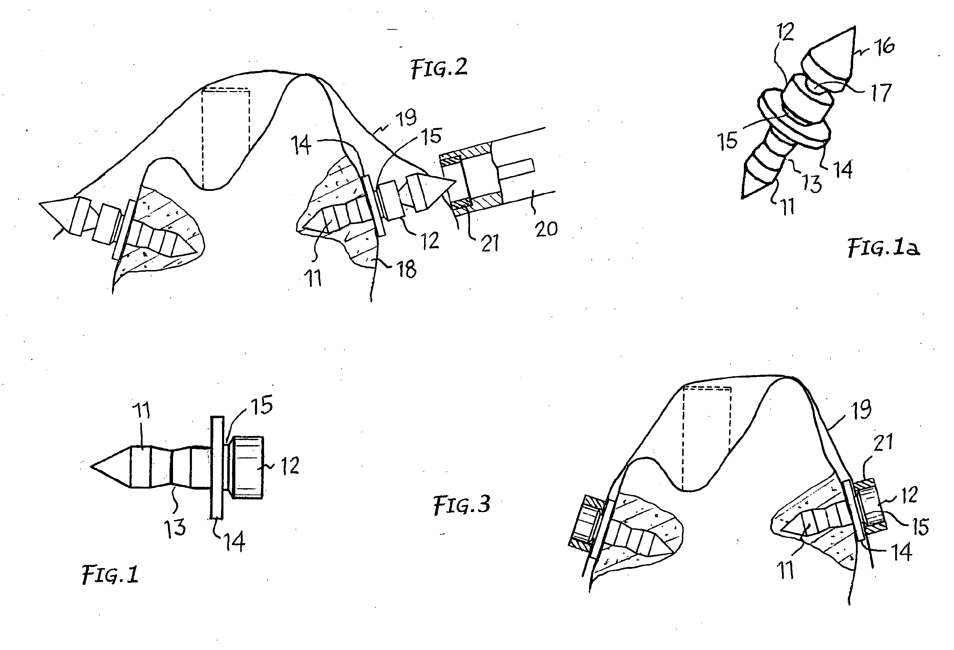

At the base of head 12, between this and the supporting flange 14, if fitted, an annular groove 15 - Fig. 1 - is obtained. At the top of head 12 or in any case within the ambit of this a means of retention is envisaged which can, as has been said above, be in the shape of a hold, of a tang or of a press stud system, for fastening and withholding a membrane 19.

In the example of embodiment shown in Figures 1a and 2 of the drawings, such a means of retention comprises a perforating portion 16, either removable or not. This perforating portion 16 can be at least in part tapered so it is pointed and, if it is removable, it joins the head 12 through a weakened breakage area 17.

As Fig. 2 shows, the micronail is fitted into the bone 18 until the flange 14 rests on this. Then a membrane 19 is positioned above the cavity of the bone to be protected, by securing it to the micronails fitted into the bone. By pressing the membrane on the perforating portion 16 of the micronail, it can be perforated in succession and moved beyond the head 12 at the level of the ring-shaped groove 15. The operation can be performed with a pushing tool 20 and with the aid of a ring or stop cap 21 associated with the tool.

When the membrane is pushed well down against the supporting flange, that part of it surrounding the perforation made settles in the ring-shaped groove where it is stably withheld by means of the ring or stop cap which is thus fitted on the head. At that point, using the same tool or with another appropriate tool, the perforating portion, if necessary, can be removed from the head of the micronail, detaching it at the level of the breakage area.

The membrane stays secured to the bone thanks to the head of the micronails to which it is fastened by means of the retention element as shown in Fig. 3.

## Claims

1. A method for fastening a protective membrane for dental use to a jaw bone, comprising, first, the fitting into the bone of a micronail having a head with at least one means of retention and, then, the fastening of the membrane to said means of retention on the head of the micronail.

2. a micronail for fastening a protective membrane to a jaw bone, comprising a shank and a head **characterised by** the fact that the head (12) has means of retention for fastening a membrane, directly or with the aid of a retention element, after the micronail has been fitted into the jaw bone involved.

3. The micronail according to claim 2, wherein the means of retention consist of a part of the head of the micronail onto which to engage the membrane, and of a retention element designed to circle or cover the head by superimposing on the membrane.

4. The micronail according to claim 2, wherein the means of retention comprise an annular groove at the base of the head and a portion of the top of the head, the membrane being withheld between said groove and said portion of the top of the head by means of a retention element circling or enclosing the head.

5. The micronail according to claim 2, wherein the head has a means of retention consisting of a tang that can be folded, after perforating and crossing the membrane, and secured by means of a retention element enclosing the membrane and the head.

6. The micronail according to any of the claims from 2, wherein the retention element consists of a ring or cap fitted to the head of the micronail.

7. The micronail according to claim 2, wherein the head has a means of retention for the membrane in the shape of a press stud.

8. The micronail according to claim 2, wherein the head has, at the top, a perforating portion (16) and, at the base, an annular groove (15) and wherein said perforating portion, after the micronail has been fitted into the bone, acts as a means for perforating and guiding the membrane towards the annular groove at the base of the head, and said groove is intended to receive and withhold part of the membrane around the hole deriving from said perforation.

9. The micronail according to claim 8, wherein said perforating portion is separable from the head after the membrane has been secured.

10. The micronail according to claim 8, wherein, with the head (12) a retention ring (21) is associated for retaining the membrane level with said annular groove, said ring being fitted after or during membrane perforation.

11. The micronail according to claims 8-10, wherein between the shank and the head a supporting flange (14) is fitted and the annular groove (15) is formed between said flange and the head.

12. The micronail according to claims 8-11, wherein the perforating portion (16) is at least partially tapered so it is pointed and joins the head through a weakened breakage part.
